# EUROPEAN PATENT SPECIFICATION

(11) **EP 0 596 553 B1**
(45) Date of publication and mention of the grant of the patent: **07.05.1997**
(21) Application number: 93202959.8
(22) Date of filing: 21.10.1993
(51) Int. Cl.: B01J 31/22, C07C 2/04, B01J 31/02, C07C 2/34

(54) **Catalyst composition for alkene oligomerisation and co-oligomerisation**
Katalytische Zusammensetzung für die Oligomerisierung und die Co-oligomerisierung von Alkene
Composition catalytique pour l'oligomérisation et la co-oligomérisation des alcènes

(30) Priority: 23.10.1992 EP 92203279
(43) Date of publication of application: 11.05.1994
(73) Proprietor: SHELL INTERNATIONALE RESEARCH MAATSCHAPPIJ B.V., 2596 HR Den Haag (NL)
(72) Inventor: Doyle, Michael John, NL-1031 CM Amsterdam (NL)

(56) References cited:
- EP-A- 0 443 686
- EP-A- 0 526 943
- WO-A-91/09882
- WO-A-92/05208
- WO-A-93/18071

## Description

This invention relates to a novel catalyst composition and to its use in the oligomerisation and co-oligomerisation of one or more alkenes, in particular alpha alkenes. More in particular, the invention relates to the oligomerisation and co-oligomerisation of ethene.

Polymerisation processes of olefins, including alkenes, such as the production of polyethylene from ethene, whereby soluble catalyst systems of the Ziegler-Natta type are used, are well known. In particular, oligomerisation processes of lower olefins to higher olefins are also well known. For example, from GB-A-1353873 it is known that C₄-C₂₀ linear alpha olefins can be prepared from ethene by oligomerisation in the presence of a nickel containing catalyst. The product linear alpha olefins, in particular those having 6-10 carbon atoms, are in great demand as intermediates in the preparation of detergents, lubricant additives and polyolefins.

However, the oligomerisation reaction also produces less valuable products, such as internal olefins and branched olefins and olefins having a number of carbon atoms outside the range of 6-24. By further processing, these by-products can be converted to the desired linear alpha olefins.

EP-A-0277003 and EP-A-0277004 both disclose catalyst compositions for the polymerisation and copolymerisation of olefins, comprising a combination of a first component which is a bis(cyclopentadienyl)titanium, zirconium or hafnium compound containing a substituent which is attached to the metal and which is capable of reacting with a cation, and a second component which is a compound having a bulky and labile anion which is substantially non-coordinating under the reaction conditions and a cation, capable of donating a proton. The two cyclopentadienyl groups may be substituted. The products according to these documents are true polymers, having a molecular weight of above 100000 (when determined in the Examples). Consequently, no attention is payed to properties which are important to olefins having a number of carbon atoms of less than 30, such as linearity and the position of the double bond.

The present Applicant's EP-A-0443686 and EP-A-0526943 both disclose methods for the co-oligomerisation of ethene with at least one other alpha olefin in the presence of a catalyst composition as broadly described above, wherein one or both of the cyclopentadienyl groups may be substituted. The preferred and only exemplified substituted ligand pair is bis-pentmethylcyclopentadienyl. The product oligomers still contain a substantial proportion of the less valuable branched olefins and internal olefins.

It has now been found that catalyst compositions as described above, wherein each of the two cyclopentadienyl radicals is differently substituted by from 1-5 hydrocarbon substituents, none of which is attached to the metal, the total number of substituents being from 2 to 9, are particularly useful in the oligomerisation or co-oligomerisation of ethene and other lower alkenes to higher alkenes, in that they affect a more preferential production of the linear alpha alkenes of the range having 4-24 carbon atoms and more in particular 6-10 carbon atoms.

Accordingly, the present invention provides a catalyst composition comprising
- a first component which is a substituted-bis(cyclopentadienyl)titanium, zirconium or hafnium compound, also containing a substituent which is attached to the metal and which is capable of reacting with a cation and
- a second component which is a compound having a bulky and labile anion containing a plurality of boron atoms which is substantially non-coordinating under the reaction conditions and a cation,
characterized in that each of the two cyclopentadienyl radicals is differently substituted by from 1 to 5 hydrocarbon substituents, none of which is attached to the metal, the total number of substituents being from 2 to 9.

Preferably, the total number of substituents is from 4 to 8.

More preferably, one of the two cyclopentadienyl radicals is mono-substituted and the other cyclopentadienyl radical is penta-substituted.

Preferably, at least one of the two cyclopentadienyl radicals contains at least one substituent which is not present on the other cyclopentadienyl radical.

The catalyst composition may be formed prior to its introduction to the reaction vessel, or it may be formed in situ.

The invention also provides the use of this catalyst composition in an oligomerisation or co-oligomerisation process, in particular in a process for the preparation of linear alpha alkenes having 4-24 carbon atoms by oligomerisation or co-oligomerisation of ethene and/or an alpha alkene having 3-10 carbon atoms.

The first component of the catalyst composition according to the invention is preferably a compound of the general formula

(Cp*)₂MR₁R₂

wherein (Cp*)₂ is a bis-cyclopentadienyl ligand pair which is differently substituted as broadly described hereinbefore, M is a metal chosen from the group of titanium, zirconium or hafnium and R₁ and R₂ are identical or different and selected from unsubstituted or substituted hydrocarbyl groups, hydrogen and halogen.

Preferably, R₁ and R₂ are alkyl groups, typically of from 1 to 5 carbon atoms, such as methyl.

The hydrocarbon substituents on the cyclopentadienyl radicals are preferably selected from the group of unsubstituted or substituted alkyls having 1-10 carbon atoms, more preferably those chosen from:
Methyl, ethyl, iso-propyl, tert-butyl, cyclohexyl, adamantyl, phenyl, ortho-tolyl, para-tolyl, 2,5-dimethylphenyl, 2-tert-butylphenyl, 2,5-di-tert-butylphenyl and mesityl.

Preferred substituted bis-cyclopentadienyl ligand pairs are:
(pentamethylcyclopentadienyl)(methylcyclopentadienyl),
(pentamethylcyclopentadienyl)(ethylcyclopentadienyl),
(pentamethylcyclopentadienyl)(iso-propylcyclopentadienyl),
(pentamethylcyclopentadienyl)(tert-butylcyclopentadienyl),
(pentamethylcyclopentadienyl)(cyclohexylcyclopentadienyl),
(pentamethylcyclopentadienyl)(adamantylcyclopentadienyl),
(pentamethylcyclopentadienyl)(phenylcyclopentadienyl),
(pentamethylcyclopentadienyl)(ortho-tolylcyclopentadienyl),
(pentamethylcyclopentadienyl)(para-tolylcyclopentadienyl),
(pentamethylcyclopentadienyl)(2,5-dimethylphenylcyclopentadienyl),
(pentamethylcyclopentadienyl)(2-tert-butylphenylcyclopentadienyl),
(pentamethylcyclopentadienyl)(2,5-di-tert-butylphenylcyclopentadienyl), and (pentamethylcyclopentadienyl)(mesitylcyclopentadienyl). Preferred metals are zirconium or hafnium.

Such complexes can be prepared for example by the routes described in "Chemistry of Organo-Zirconium and Hafnium Compounds", by Lappert et al., 1986, published by John Wiley & Sons, Chichester, England.

The second component of the catalyst composition according to the invention is an ionic combination of a bulky anion containing a plurality of boron atoms and a proton-donating cation, the anion being such that it is substantially non-coordinating under the reaction conditions employed. Thus, it is intended that the anion should not coordinate, or at least coordinate only weakly, to the bis(cyclopentadienyl) metal entity of the first component. The boron-containing non-coordinating anion is preferably a carborane anion, suitably a carborane anion of the formula B₁₁CH₁₂⁻. Such carboranes are known and can be prepared by methods such as that of Shelly et al, J. Am. Chem. Soc. 107 (1985) 5955-5959. Other bulky boron containing anions may also be used, such as a tetra(perfluorophenyl) boron anion. The proton-donating cation is preferably a quaternary ammonium cation such as a trialkylammonium cation, for example tri-n-butylammonium cation. Alternatively a cation may be used which is not proton-donating, such as a metal cation e.g. a silver ion, or a triphenylcarbenium ion.

The catalyst composition may be formed by mixing together the two components, preferably in solution in a suitable solvent such as toluene, chlorobenzene, an alkane or an alkene, to form a liquid catalyst system. The two components are generally employed in substantially equimolar amounts, although the molar ratio of the first component to the second component may vary within the range of from 0.1 to 5.0. Such a quantity of the catalyst system is usually employed in the reaction mixture as to contain from 10⁻¹ to 10⁻⁷ gram atoms, in particular from 10⁻³ to 10⁻⁵ gram atoms, of the metal per mole of olefin to be reacted.

The oligomerisation reaction according to the invention can be carried out in batch or continuous operation.

The oligomerisation reaction is generally, although not necessarily, carried out in an inert liquid which is suitably also the solvent for the catalyst components. The reaction is suitably carried out at an elevated temperature, preferably in the range of from 20 to 175 °C, more preferably at 50 to 150 °C. The reaction is suitably carried out under conditions of moderately elevated pressure, preferably in the range of from 100 to 10000 kPa, more preferably from 500 to 6000 kPa. The optimum conditions of temperature and pressure used in a particular reaction system in order to maximise the yield of the desired linear alpha alkenes can be readily established by those skilled in the art, but it has been found that conditions of between 70-90 °C and between 4000-6000 kPa are particularly advantageous in this respect with the catalyst systems of the present invention.

The starting reactants may be supplied to the reactor together with an inert diluent, such as nitrogen or helium when the reactant is gaseous, and a liquid solvent, e.g. the same solvent as that of the catalyst components, when the reactant is in the liquid form.

The reaction is preferably carried out in the absence of air or moisture.

Reaction times of from 1 minute to 5 hours have been found to be suitable, depending on the activity of the catalyst system and on the reaction conditions. After a suitable reaction time, a conventional catalyst deactivating agent such as water, methanol, or another alcohol, may be added if desired to the reaction mixture in order to terminate the reaction. Alternatively, the reaction can simply be terminated by the introduction of air.

The product mixed alkenes are preferentially linear alpha alkenes having a chain length within the range of 5 to 24 carbon atoms, of which those having between 6 and 10 carbon atoms in the chain are particularly preferred. They may be suitably recovered by distillation and separation techniques known in the art. If desired, unconverted starting material and oligomeric products having a molecular weight outside the desired molecular weight may be recovered, processed if necessary and recycled to be used as starting material in a subsequent oligomerisation reaction.

The invention will be further illustrated by the following examples.

### Examples 1 to 7

The procedures were carried out with rigorous exclusion of oxygen and moisture.

Catalyst liqours were prepared by dissolving in toluene, as First Component, in 30 ml, 0.25 mmol of (Cp*)₂MR₂, wherein M is zirconium, R₂ is dimethyl and (Cp*)₂ is:
in comparative Example 1: bis-pentamethylcyclopentadienyl;
in comparative Example 2: bis-methylcyclopentadienyl;
in comparative Example 3: bis-iso-propylcyclopentadienyl;
in comparative Example 4: bis-tert-butylcyclopentadienyl;
in Example 5: (pentamethylcyclopentadienyl)(methylcyclopentadienyl);
in Example 6: (pentamethylcyclopentadienyl)(iso-propylcyclopentadienyl);
in Example 7: (pentamethylcyclopentadienyl)(tert-butylcyclopetadienyl);
and as Second Component, in 70 ml, in all cases 0.05 mmol of Bu₃NHB₁₁CH₁₂ (tri-n-butylammonium 1-carbadodecarborate).

The Second Component of the catalytic composition was first added to an autoclave of 500 ml, followed by pressurising the autoclave with ethene to 1000 kPa, and heating to 90 °C.

Subsequently, the reaction was started by adding the First Component of the catalytic composition. Pressure was maintained at 1000 kPa during the reaction by continuously recharging of consumed ethene.

At the end of the (predetermined) reaction time, the reaction was terminated by water injection. The product distribution was determined by gas-liquid chromatography.

The amount of ethene consumed during the reaction, the amounts of C₄, C₆-C₁₀ and C₁₂₊ olefins produced, and the wt% distribution of alpha-, beta- and branched hexene are given in Table 1.

**TABLE 1**

| Example | 1 | 2 | 3 | 4 | 5 | 6 | 7 |
|---|---|---|---|---|---|---|---|
| Reaction time (min) | 3 | 103 | 70 | 150 | 25 | 26 | 20 |
| Ethene consumed (g) | 25 | 25 | 25 | 25 | 25 | 25 | 25 |
| Product C₄ olefin (g) | 0.9 | 2.0 | 4.0 | 22.4 | 2.2 | 6.0 | 17.8 |

| Product C₆₋₁₀ | | | | | | | |
|---|---|---|---|---|---|---|---|
| olefins (g) | 3.8 | 7.0 | 10.8 | 2.6 | 7.5 | 12.5 | 7.1 |
| hexene (g) | 1.1 | 2.3 | 4.1 | 2.4 | 2.5 | 5.3 | 5.3 |
| Product C₁₂₊ | | | | | | | |
| olefins (g) | 20.3 | 16.0 | 10.2 | 0.0 | 15.3 | 6.6 | 0.1 |

| Distribution of hexenes (wt%) | | | | | | | |
|---|---|---|---|---|---|---|---|
| 1-hexene | 81.8 | 57.9 | 61.2 | 44.3 | 96.6 | 95.9 | 89.1 |
| 2-hexene | 17.6 | 39.4 | 36.6 | 15.4 | 3.0 | 3.2 | 1.9 |
| 2-ethyl-1-butene | 0.7 | 2.7 | 2.2 | 40.3 | 0.4 | 0.9 | 9.0 |

### Examples 8 to 10

The procedure was the same as that of Examples 1 to 7, except that,
- as First Component, 1 mmol (Example 8 and 9) or 0.25 mmol (Example 10) of (Cp*)₂MR₂ was used, wherein M is hafnium, R₂ is dimethyl and (Cp*)₂ is:
   in comparative Example 8: bis-pentamethylcyclopentadienyl;
   in comparative Example 9: bis-tert-butylcyclopentadienyl;
   in Example 10: (pentamethylcyclopentadienyl)(tert-butylcyclopetadienyl);
- as Second Component, 1 mmol (Example 8 and 9) or 0.1 mmol (Example 10) of Bu₃NHB₁₁CH₁₂ (tri-n-butylammonium 1-carbadodecacarborate) was used; and the total amount of toluene solvent in the autoclave was 270 ml (Example 8 and 9) or 70 ml (Example 10).

The amount of ethene consumed during the reaction, the amounts of C₄, C₆-C₁₀ and C₁₂₊ olefins produced, and the wt% distribution of alpha-, beta- and branched hexene are given in Table 2.

**TABLE 2**

| Example | 8 | 9 | 10 |
|---|---|---|---|
| Reaction time (min) | 2 | 30 | 30 |
| Ethene consumed (g) | 40 | 76 | 30 |
| Product C₄ olefin (g) | 1.6 | 72.2 | 23.4 |
| Product C₆₋₁₀ olefins (g) | 6.7 | 2.3 | 6.5 |
| hexene (g) | 2.0 | 2.2 | 5.3 |
| Product C₁₂₊ olefins (g) | 31.7 | 1.5 | 0.1 |

| Distribution of hexenes (wt%) | | | |
|---|---|---|---|
| 1-hexene | 23.6 | 22.6 | 76.2 |
| 2-hexene | 75.9 | 2.4 | 0.8 |
| 2-ethyl-1-butene | 0.5 | 75.0 | 23.0 |

### Examples 11 to 13

The procedure was the same as that of comperative Examples 1 and 2 and Example 5, respectively, except that the First and Second Components of the catalyst composition where dissolved in 15 and 60 ml of toluene respectively and that, together with the Second Component, 210 mmol of 1-pentene (co-monomer for reacting with the ethene) was injected into the autoclave.

The amount of ethene consumed during the reaction, the amounts of C₄, C₆₊₈₊₁₀, C₇₊₉₊₁₁ and C₁₂₊ olefins produced, and the wt% distribution of alpha-, beta- and branched hexene and heptene are given in Table 3.

**TABLE 3**

| Example | 11 | 12 | 13 |
|---|---|---|---|
| Reaction time (min) | 4 | 150 | 38 |
| Ethene consumed (g) | 25 | 25 | 25 |
| Product C₄ olefin (g) | 0.2 | 0.8 | 2.5 |
| Product C₆₊₈₊₁₀ olefins (g) | 0.8 | 2.9 | 7.5 |
| hexene (g) | 0.2 | 1.0 | 2.6 |
| Product C₇₊₉₊₁₁ olefins (g) | 4.5 | 2.7 | 3.8 |
| heptene (g) | 1.4 | 1.0 | 1.4 |
| Product C₁₂₊ olefins (g) | 24.5 | 21.6 | 15.2 |

| Distribution of hexenes (wt%) | | | |
|---|---|---|---|
| 1-hexene | 89.8 | 60.3 | 96.9 |
| 2-hexene | 10.2 | 38.9 | 2.7 |
| 2-ethyl-1-butene | 0.0 | 0.8 | 0.4 |

| Distribution of heptenes (wt%) | | | |
|---|---|---|---|
| 1-heptene | 86.3 | 57.6 | 93.1 |
| 2-heptene | 13.4 | 31.6 | 2.7 |
| 2-ethyl-1-pentene | 0.3 | 10.8 | 4.2 |

From these Examples it is apparent, that catalyst compositions according to the invention, when used in the oligomerisation of ethene (Examples 5-7 and 10) and in the co-oligomerisation of ethene and pentene (Example 13), have the advantage above comparative catalyst compositions of promoting the selectivity of the reaction towards linear alpha-olefin reaction products.

## Claims

1. A catalyst composition comprising a first component which is a substituted-bis(cyclopentadienyl) titanium, zirconium or hafnium compound, also containing a substituent which is attached to the metal and which is capable of reacting with a cation and a second component which is a compound having a bulky and labile anion containing a plurality of boron atoms which is substantially non-coordinating under the reaction conditions and a cation,
characterized in that each of the two cyclopentadienyl radicals is differently substituted by from 1 to 5 hydrocarbon substituents, none of which is attached to the metal, the total number of substituents being from 2 to 9.

2. A catalyst composition according to claim 1, characterized in that one of the two cyclopentadienyl radicals is mono-substituted and the other cyclopentadienyl radical is penta-substituted.

3. A catalyst composition according to claim 1 or 2, characterized in that least one of the two cyclopentadienyl radicals contains at least one substituent which is not present on the other cyclopentadienyl radical.

4. A catalyst composition according to any one of claims 1-3, characterized in that the first component of the catalyst is a compound of the general formula
(Cp*)₂MR₁R₂
wherein (Cp*)₂ is a bis-cyclopentadienyl ligand pair which is differently substituted as defined hereinbefore, M is a metal chosen from the group of titanium, zirconium or hafnium and R₁ and R₂ are two identical or different groups selected from unsubstituted or substituted hydrocarbyl groups, alkyloxy groups, hydrogen or halogen.

5. A catalyst composition according to claim 4, characterized in that R₁ and R₂ are unsubstituted alkyl groups of from 1 to 5 carbon atoms.

6. A catalyst composition according to any one of claims 1-5, characterized in that the hydrocarbon substituents on the cyclopentadienyl radicals are chosen from the group of: unsubstituted or substituted alkyls or aryls having 1-10 carbon atoms, preferably from methyl, ethyl, iso-propyl, tert-butyl, cyclohexyl, adamantyl, phenyl, ortho-tolyl, para-tolyl, 2,5-dimethylphenyl, 2-tert-butylphenyl, 2,5-di-tert-butylphenyl and mesityl.

7. A catalyst composition according to any one of claims 1-6, characterized in that the substituted bis-cyclopentadienyl ligand pair is chosen from the group of:
(pentamethylcyclopentadienyl)(methylcyclopentadienyl),
(pentamethylcyclopentadienyl)(ethylcyclopentadienyl),
(pentamethylcyclopentadienyl)(iso-propylcyclopentadienyl),
(pentamethylcyclopentadienyl)(tert-butylcyclopentadienyl),
(pentamethylcyclopentadienyl)(cyclohexylcyclopentadienyl),
(pentamethylcyclopentadienyl)(adamantylcyclopentadienyl),
(pentamethylcyclopentadienyl)(phenylcyclopentadienyl),
(pentamethylcyclopentadienyl)(ortho-tolylcyclopentadienyl),
(pentamethylcyclopentadienyl)(para-tolylcyclopentadienyl),
(pentamethylcyclopentadienyl)(2,5-dimethylphenylcyclopentadienyl),
(pentamethylcyclopentadienyl)(2-tert-butylphenylcyclopentadienyl),
(pentamethylcyclopentadienyl)(2,5-di-tert-butylphenylcyclopentadienyl), and (pentamethylcyclopentadienyl)(mesitylcyclopentadienyl).

8. A catalyst composition according to any one of claims 1-7, characterized in that the boron-containing non-coordinating anion is a carborane anion.

9. A catalyst composition according to claim 8, characterized in that the carborane anion is of the formula B₁₁CH₁₂⁻.

10. A catalyst composition according to any one of claims 1-9, characterized in that the cation is a proton-donating cation.

11. A catalyst composition according to claim 10, characterized in that the proton-donating cation is a quaternary ammonium cation.

12. A catalyst according to any one of claims 1-9, characterized in that the cation is a silver ion.

13. Use of a catalyst composition as claimed in any one of claims 1-12 in a process for the preparation of linear alpha alkenes having 4-24 carbon atoms by oligomerisation or co-oligomerisation of ethene and/or an alpha alkene having 3-10 carbon atoms.

## Patentansprüche

1. Katalysatorzusammensetzung, enthaltend eine erste Komponente, bei der es sich um eine Bis(cyclopentadienyl)titan-, -zirkonium- oder -hafniumverbindung mit substituierten Cyclopentadienylresten, die daneben auch einen an das Metall gebundenen Substituenten, der mit einem Kation reagieren kann, enthält, handelt, und eine zweite Komponente, bei der es sich um eine Verbindung mit einem sperrigen und labilen Anion mit mehreren Boratomen, das unter den Reaktionsbedingungen im wesentlichen nichtkoordinierend ist, und einem Kation handelt,
dadurch gekennzeichnet, daß jeder der beiden Cyclopentadienylreste verschieden mit 1 bis 5 Kohlenwasserstoffsubstituenten, von denen keiner an das Metall gebunden ist, substituiert ist, wobei die Gesamtzahl der Substituenten 2 bis 9 beträgt.

2. Katalysatorzusammensetzung nach Anspruch 1, dadurch gekennzeichnet, daß einer der beiden Cyclopentadienylreste monosubsituiert und der andere Cyclopentadienylrest pentasubstituiert ist.

3. Katalysatorzusammensetzung nach Anspruch 1 oder 2, dadurch gekennzeichnet, daß mindestens einer der beiden Cyclopentadienylreste mindestens einen Substituenten enthält, den der andere Cyclopentadienylrest nicht trägt.

4. Katalysatorzusammensetzung nach einem der Ansprüche 1-3, dadurch gekennzeichnet, daß es sich bei der ersten Komponente des Katalysators um eine Verbindung der allgemeinen Formel
(Cp*)₂MR₁R₂
handelt, worin (Cp*)₂ ein wie oben beschrieben unterschiedlich substituiertes Biscyclopentadienylligandenpaar, M ein Metall, ausgewählt aus der Gruppe Titan, Zirkonium oder Hafnium, und R₁ und R₂ gleiche oder verschiedene Gruppen, ausgewählt aus gegebenenfalls substituierten Kohlenwasserstoffgruppen, Alkoxygruppen, Wasserstoff oder Halogen, bedeuten.

5. Katalysatorzusammensetzung nach Anspruch 4, dadurch gekennzeichnet, daß R₁ und R₂ unsubstituierte Alkylgruppen mit 1 bis 5 Kohlenstoffatomen bedeuten.

6. Katalysatorzusammensetzung nach einem der Ansprüche 1-5, dadurch gekennzeichnet, daß die Kohlenwasserstoffsubstituenten an den Cyclopentadienylresten aus folgender Gruppe ausgewählt sind: gegebenenfalls substituierte Alkyle oder Aryle mit 1-10 Kohlenstoffatomen, bevorzugt aus Methyl, Ethyl, Isopropyl, tert.-Butyl, Cyclohexyl, Adamantyl, Phenyl, ortho-Tolyl, para-Tolyl, 2,5-Dimethylphenyl, 2-tert.-Butylphenyl, 2,5-Di-tert.-butylphenyl und Mesityl.

7. Katalysatorzusammensetzung nach einem der Ansprüche 1-6, dadurch gekennzeichnet, daß das substituierte Biscyclopentadienylligandenpaar aus folgender Gruppe ausgewählt ist:
(Pentamethylcyclopentadienyl)(methylcyclopentadienyl), (Pentamethylcyclopentadienyl)(ethylcyclopentadienyl), (Pentamethylcyclopentadienyl)(isopropylcyclopentadienyl), (Pentamethylcyclopentadienyl)(tert.-butylcyclopentadienyl), (Pentamethylcyclopentadienyl)(cyclohexylcyclopentadienyl), (Pentamethylcyclopentadienyl)(adamantylcyclopentadienyl), (Pentamethylcyclopentadienyl)(phenylcyclopentadienyl), (Pentamethylcyclopentadienyl)(ortho-tolylcyclopentadienyl), (Pentamethylcyclopentadienyl)(para-tolylcyclopentadienyl), (Pentamethylcyclopentadienyl)(2,5-dimethylphenylcyclopentadienyl), (Pentamethylcyclopentadienyl)(2-tert.-butylphenylcyclopentadienyl), (Pentamethylcyclopentadienyl)(2,5-di-tert.-butylphenylcyclopentadienyl) und (Pentamethylcyclopentadienyl)(mesitylcyclopentadienyl).

8. Katalysatorzusammensetzung nach einem der Ansprüche 1-7, dadurch gekennzeichnet, daß es sich bei dem borhaltigen, nichtkoordinierenden Anion um ein Carboran-Anion handelt.

9. Katalysatorzusammensetzung nach Anspruch 8, dadurch gekennzeichnet, daß das Carboran-Anion die Formel B₁₁CH₁₂⁻ hat.

10. Katalysatorzusammensetzung nach einem der Ansprüche 1-9, dadurch gekennzeichnet, daß es sich bei dem Kation um ein protonenabgebendes Kation handelt.

11. Katalysatorzusammensetzung nach Anspruch 10, dadurch gekennzeichnet, daß es sich bei dem protonenabgebenden Kation um ein quaternäres Ammoniumkation handelt.

12. Katalysatorzusammensetzung nach einem der Ansprüche 1-9, dadurch gekennzeichnet, daß es sich bei dem Kation um ein Silberion handelt.

13. Verwendung einer Katalysatorzusammensetzung gemäß einem der Ansprüche 1-12 bei einem Verfahren zur Herstellung von linearen alpha-Alkenen mit 4-24 Kohlenstoffatomen durch Oligomerisierung oder Cooligomerisierung von Ethen und/oder einem alpha-Alken mit 3-10 Kohlenstoffatomen.

## Revendications

1. Composition catalytique comprenant
- un premier composant qui est un composé d'hafnium, de zirconium, ou de bis(cyclopentadiényl)titane substitué, contenant également un substituant qui est attaché au métal et qui est capable de réagir avec un cation et
- un second composant qui est un composé possédant un anion volumineux et labile contenant une multiplicité d'atomes de bore, qui n'est sensiblement pas coordinent dans les conditions réactionnelles et un cation,
caractérisée en ce que chacun des deux radicaux cyclopentadiényle est différemment substitué par de 1 à 5 substituants hydrocarbonés, aucun d'entre eux n'étant attaché au métal, le nombre total des substituants variant de 2 à 9.

2. Composition catalytique suivant la revendication 1, caractérisée en ce que l'un des deux radicaux cyclopentadiényle est monosubstitué, cependant que l'autre radical cyclopentadiényle est pentasubstitué.

3. Composition catalytique suivant la revendication 1 ou 2, caractérisée en ce qu'au moins l'un des deux radicaux cyclopentadiényle contient au moins un substituant qui n'est présent sur l'autre radical cyclopentadiényle.

4. Composition catalytique suivant l'une quelconque des revendications 1 à 3, caractérisée en ce que le premier composant du catalyseur est un composé de la formule générale
(Cp*)₂MR₁R₂
dans laquelle (Cp*)₂ est une paire de ligands du type bis-cyclopentadiényle qui sont différemment substitués, de la manière précédemment définie, M est un métal choisi parmi le titane, le zirconium et l'hafnium est les symboles R₁ et R₂ ont des significations identiques ou différentes et représentent des radicaux hydrocarbyle non substitué ou substitués, des radicaux alkyloxy, des atomes d'hydrogène, ou des atomes d'halogènes.

5. Composition catalytique suivant la revendication 4, caractérisée en ce que R₁ et R₂ représentent des radicaux alkyle non substitués, qui comportent de 1 à 5 atomes de carbone.

6. Composition catalytique suivant l'une quelconque des revendications 1 à 5, caractérisée en ce que les substituants hydrocarbonés sur les radicaux cyclopentadiényle sont choisis dans le groupe formé par les radicaux alkyle ou aryle, non substitués ou substitués, possédant de 1 à 10 atomes de carbone, de préférence, parmi les groupes suivants :
méthyle, éthyle, isopropyle, tert-butyle, cyclohexyle, adamantyle, phényle, ortho-tolyle, para-tolyle, 2,5-diméthylphényle, 2-tert-butylphényle, 2,5-di-tert-butylphényle et mésityle.

7. Composition catalytique suivant l'une quelconque des revendications 1 à 6, caractérisée en ce que la paire de ligands du type bis-cyclopentadiényle substitués est choisie dans le groupe formé par celles qui suivent :
(pentaméthylcyclopentadiényl)(méthylcyclopentadiényle),
(pentaméthylcyclopentadiényl)(éthylcyclopentadiényle),
(pentaméthylcyclopentadiényl)(isopropylcyclopentadiényle),
(pentaméthylcyclopentadiényl)(tert-butylcyclopentadiényle),
(pentaméthylcyclopentadiényl)(cyclohexylcyclopentadiényle),
(pentaméthylcyclopentadiényl)(adamantylcyclopentadiényle),
(pentaméthylcyclopentadiényl)(phénylcyclopentadiényle),
(pentaméthylcyclopentadiényl)(ortho-tolylcyclopentadiényle),
(pentaméthylcyclopentadiényl)(para-tolylcyclopentadiényle),
(pentaméthylcyclopentadiényl)(2,5-diméthylphénylcyclopentadiényle),
(pentaméthylcyclopentadiényl)(2-tert-butylphénylcyclopentadiényle),
(pentaméthylcyclopentadiényl)(2,5-di-tert-butylphénylcyclopentadiényle), et
(pentaméthylcyclopentadiényl)(mésitylcyclopentadiényle).

8. Composition catalytique suivant l'une quelconque des revendications 1 à 7, caractérisée en ce que l'anion non coordinent contenant du bore est un anion carborane.

9. Composition catalytique suivant la revendication 8, caractérisée en ce que l'anion carborane répond à la formule B₁₁CH₁₂⁻.

10. Composition catalytique suivant l'une quelconque des revendications 1 à 9, caractérisée en ce que le cation est un cation donneur de protons.

11. Composition catalytique suivant la revendication 10, caractérisée en ce que le cation donneur de protons est un cation ammonium quaternaire.

12. Composition catalytique suivant l'une quelconque des revendications 1 à 9, caractérisée en ce que le cation est un ion argent.

13. Utilisation d'une composition catalytique suivant l'une quelconque des revendications 1 à 12, pour la mise en oeuvre d'un procédé de préparation d'alpha-alcènes linéaires comportant de 4 à 24 atomes de carbone, par l'oligomérisation ou la co-oligomérisation de l'éthène et/ou d'un alpha-alcène, possédant de 3 à 10 atomes de carbone.
